# EUROPEAN PATENT SPECIFICATION

(11) **EP 2 501 688 B1**
(45) Date of publication and mention of the grant of the patent: **16.10.2013**
(21) Application number: 10779589.0
(22) Date of filing: 12.11.2010
(51) Int. Cl.: C07D 301/12

(54) **EPOXIDIZING OF PROPYLENE**
EPOXIDIERUNG VON PROPYLEN
ÉPOXYDATION DE PROPYLÈNE

(30) Priority: 19.11.2009 US 281547 P
(43) Date of publication of application: 26.09.2012
(73) Proprietor: Dow Global Technologies LLC, Midland, MI 48674 (US)
(72) Inventor: CRAMPTON, Hannah, L., Lake Jackson, TX (US)
(74) Representative: Raynor, John
(86) International application number: PCT/US2010/002966
(87) International publication number: WO 2011/062609

(56) References cited:
- EP-A1- 1 085 017

## Description

### Field of Invention

This invention relates to a process for epoxidizing propylene to produce propylene oxide.

### Background

Epoxides are produced by a variety of techniques. One commercial technique for the production of epoxides includes reacting an olefin with hydrogen peroxide and one or more catalysts in a protic medium. The epoxidation of olefins in a protic medium, however, can decrease the selectivity of the epoxidation reaction. In addition to a decrease in selectivity, the amount of by-products formed during the epoxidation can increase as the epoxide reacts with the protic medium and as the epoxide oligomerizes and/or polymerizes. This decrease in selectivity also increases the costs of production because of a lower yield of epoxides and the steps required to separate the by-products from the epoxide.

As such, efforts have been made to increase the selectivity of the epoxidation process. Such efforts include using pre-treated catalysts and homogeneous organic or inorganic compounds to change the pH of the reaction mixture. However, while the selectivity of the epoxidation reaction may increase in these previous approaches, the hydrogen peroxide utilization, hydrogen peroxide conversion and the lifetime of the catalyst can decrease simultaneously. These drawbacks decrease the efficiency of the production by yielding less epoxide for the amount of materials used.

### Summary

The present invention provides one or more embodiments of a process for epoxidizing propylene. For the embodiments, epoxidizing propylene includes reacting propylene with a hydrogen peroxide solution at a predetermined pH iri the presence of a catalyst and a solvent at a predetermined reaction temperature. For the embodiments, a pH of the hydrogen peroxide solution is adjusted to the predetermined pH by contacting the hydrogen peroxide solution with a supported base to remove acidic species from the hydrogen peroxide solution. For the embodiments, a selectivity of an epoxidation of propylene is increased without decreasing a hydrogen peroxide utilization or a hydrogen peroxide conversion as compared to an epoxidation of propylene without the supported base to adjust the pH of the hydrogen peroxide solution to the predetermined pH.

### Brief Description of the Drawings

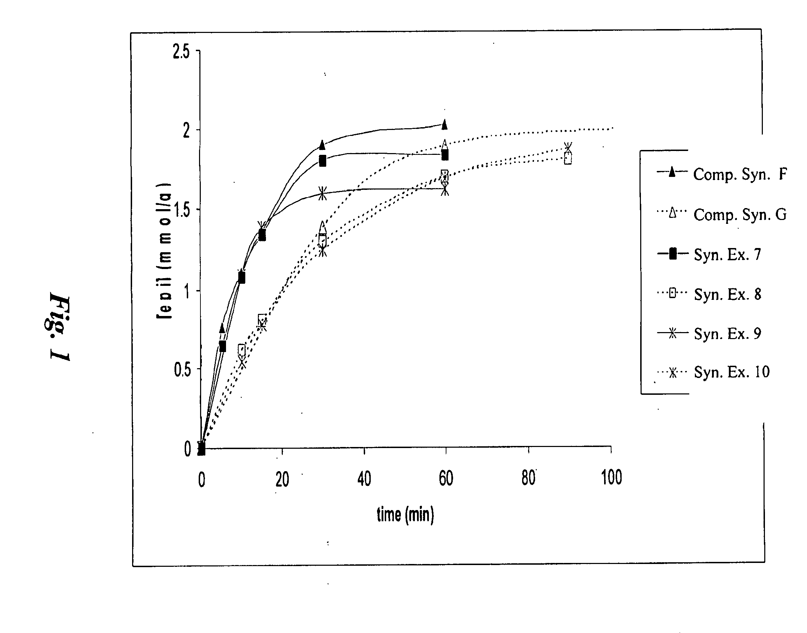

Figure 1 illustrates the amount of epichlorohydrin formed during an epoxidation of allyl chloride.

### Detailed Description

### Definitions

"Epoxide" refers to a compound in which an oxygen atom is directly attached to two adjacent or non-adjacent carbon atoms of a carbon chain or ring system. Propylene oxide is an example of an epoxide and formed from the epoxidation of propylene.

"Selectivity" refers to the amount of propylene oxide formed relative the amount of propylene oxide formed plus all by-products.

"Reaction mixture" refers to a mixture of propylene, a hydrogen peroxide solution at a predetermined pH, a catalyst and a solvent. The reaction mixture can further include additional reagents including, but not limited to, a co-solvent that is more fully discussed herein.

"By-products" refers to all substances formed from epoxidizing propylene minus the propylene oxide. For example, for an epoxidation of propylene, the by-products can include water, propanediol, methoxypropanols and higher molecular weight by-products.

"Higher molecular weight by-products" refer to the by-products formed from epoxidizing propylene that elute after propanediol and methoxypropanols during gas chromatography.

"Hydrogen peroxide conversion" refers to the amount of hydrogen peroxide that reacts during an epoxidation of proplyene relative the amount of hydrogen peroxide added to the reaction mixture.

"Hydrogen peroxide utilization" refers to the amount of hydrogen peroxide converted to propylene oxide relative the amount of hydrogen peroxide that reacts during the epoxidation of propylene.

"Supported base" refers to a non-soluble support bearing a basic functional group that has a neutral charge.

"Neutral charge" refers to a substance that does not have ions such that the substance has neither a positive nor negative electric charge.

"Stabilizer" refers to substances, and in particular acid(s), that include acidic species that are added to a hydrogen peroxide solution to reduce the rate of decomposition.

"Acidic species" refers to a substance that can donate a proton.

For the embodiments, a process for epoxidizing propylene of the present invention (also referred to as "epoxidation process") includes reacting an olefin, such as propylene, with a hydrogen peroxide solution at a predetermined pH in the presence of a catalyst and a solvent at a predetermined reaction temperature. For the embodiments, a pH of the hydrogen peroxide solution is adjusted to the predetermined pH by contacting the hydrogen peroxide solution with a supported base to remove acidic species from the hydrogen peroxide solution. The epoxidation process of the present invention increases a selectivity of an epoxidation of propylene without decreasing a hydrogen peroxide utilization or a hydrogen peroxide conversion as compared to the epoxidation of propylene without the use of the supported base to adjust the pH of the hydrogen peroxide solution to the predetermined pH.

For the embodiments, the epoxidation process includes reacting hydrogen peroxide with the olefin. The hydrogen peroxide is in an aqueous solution, referred to as a hydrogen peroxide solution, where the hydrogen peroxide in the hydrogen peroxide solution reacts with the olefin to produce the epoxide. The hydrogen peroxide solution can further contain other substances that may or may not participate in the epoxidation reaction that forms the epoxide. For example, acidic species can be present in the hydrogen peroxide solution and are discussed more fully herein.

For the embodiments, the olefin used in the present invention is propylene, and is epoxidized to propylene oxide. The amount of propylene used in the reaction mixture can be within a range of from 10 weight percent (wt%) to 90 wt%, more preferably within a range of from 30 wt% to 70 wt%, and still more preferably within a range of from 40 wt% to 65 wt%, based on the total weight of the reaction mixture.

For the embodiments, the epoxidation process includes reacting propylene with hydrogen peroxide, where the hydrogen peroxide is in the hydrogen peroxide solution. However, as one skilled in the art would appreciate, other organic and/or inorganic hydroperoxides may be used for the epoxidation of propylene. Examples of other hydroperoxides that may be used include, but are not limited to, tert-butyl hydroperoxide, ethylbenzene hydroperoxide, acetyl peroxide, benzoyl peroxide, methyl ethyl ketone peroxide, cumene peroxide and combinations thereof. For the embodiments, the amount of the hydrogen peroxide solution used in the reaction mixture can be within a range of from 1 wt% to 35 wt%, more preferably within a range of from I wt% to 15 wt%, and still more preferably within a range of from 1 wt% to 7 wt%, based on the total weight of the reaction mixture.

Available sources of the hydrogen peroxide solution are produced by the hydrolysis of a persulphuric acid and, more commonly, the successive hydrogenation and oxidation of a substituted alkylanthroquinone in a suitable solvent system. Both methods produce a hydrogen peroxide solution that can contain high levels of impurities such as solids and transition metal ions that are introduced during the production of the hydrogen peroxide solution. Hydrogen peroxide solutions with even trace levels of impurities tend to decompose during storage and/or use. Therefore, stabilizers, which include acidic species, are added to the hydrogen peroxide solution to reduce and/or prevent decomposition. Examples of stabilizers can include phosphoric acid, nitric acid, tin, stannates, and organic phosphates, or mixtures thereof.

For the embodiments, the epoxidation process includes adjusting a pH of the hydrogen peroxide solution to a predetermined pH prior to reacting the hydrogen peroxide solution with propylene. The pH of the hydrogen peroxide solution is adjusted by contacting the hydrogen peroxide solution with a supported base. The supported base is selected from supported bases that have a predominantly neutral charge and do not participate in an ion exchange. In other words, the supported base does not donate an ion in exchange for another ion, but rather can either accept or donate ions, but not both. For the embodiments, the supported base can act like a "destabilizer" and reduce the acidic species and metals that may be present from the production of the hydrogen peroxide solution. The supported base can reduce acidic species present in the hydrogen peroxide solution by accepting ions, but does not donate ions in return. More specifically, the supported base can accept protons. By accepting the protons, the predominately neutral charge of the supported base will change to a positive charge while the pH of the hydrogen peroxide solution is adjusted to the predetermined pH.

For the embodiments, the amount of the supported base needed to adjust the pH of the hydrogen peroxide solution to the predetermined pH will depend on the amount of hydrogen peroxide solution being used in the reaction mixture and the predetermined pH value. Therefore, enough of the supported base is used until the predetermined pH of the hydrogen peroxide solution is achieved. For the embodiments, the predetermined pH can be within a range of from 1.0 to 9.0, more preferably within a range of from 3.0 to 8.0, and still more preferably within a range of from 6.0 to 8.0.

As discussed herein, the epoxidation process of the present invention can reduce the amount of by-products formed during the epoxidation of propylene. The epoxidation of propylene produces by-products that can include water, propanediol, methoxypropanols, and higher molecular weight by-products. These by-products can be formed by the stabilizers present in the hydrogen peroxide solution. For example, the acidic species in the stabilizers can catalyze a ring opening reaction during the formation of propylene oxide. These ring opening reactions can produce the higher molecular weight by-products and a portion of the propanediol and methoxypropanols. Therefore, reducing the acidic species in the hydrogen peroxide solution limits the ring opening reaction and reduces the amount of by-products formed during the epoxidation. By limiting the ring opening reactions, the selectivity of the epoxidation of propylene is increased and more propylene oxide is produced during the epoxidation. For the embodiments of the present invention, increasing the selectivity is accomplished without decreasing the hydrogen peroxide utilization or the hydrogen peroxide conversion as compared to the epoxidation of propylene without the supported base to adjust the pH of the hydrogen peroxide solution to a predetermined pH.

For the embodiments, the pH of the hydrogen peroxide solution is adjusted to the predetermined pH by contacting the hydrogen peroxide solution with the supported base. Contacting the hydrogen peroxide solution with the supported base can be performed in either a batch, semi-continuous or continuous mode. For example, the hydrogen peroxide solution can be mixed with the supported base to form a heterogeneous solution or the hydrogen peroxide solution can be passed through a fixed-bed reactor that contains the supported base. For the embodiments, mixing the hydrogen peroxide solution with the supported base may be performed by known means for mixing, such as, but not limited to, stirring with an agitator or by inducing shear with a mixing element in a tubular reactor or loop reactor. Additionally, using combinations of the reactors to contact the hydrogen peroxide solution may also be used.

For the embodiments, epoxidizing propylene is carried out in the presence of a catalyst. Additionally, more than one catalyst may be used in the epoxidation process. The catalyst used can be selected from heterogeneous catalysts which comprise a porous oxide material such as zeolite. As appreciated, zeolites are solid containing silicas which have microporous crystalline ordered channels with a cage structure and pore openings. Along with microporous zeolites, mesoporous and macroporous zeolite type catalysts can also be used. For the embodiments, the catalyst is preferably selected from titanium-silicalites generally known as TS-1 having a MFI structure. It is also possible to use titanium-silicalites with a MEL or intermediate MFI/MEL structure and titanium-silicalites from beta zeolites containing titanium and having a BEA structure. Other titanium containing zeolite catalysts generally known as TS-2, TS-3, ZSM-48 and ZMS-12 may also be used.

For the embodiments, a portion or all of the titanium in the zeolite catalyst can be replaced by, but not limited to, boron, aluminum, iron, gallium, vanadium, zirconium, chromium, niobium or a mixture of two or more thereof. Additional examples of zeolites containing titanium, vanadium, chromium, niobium, and zirconium include, but are not limited to, BEA, MOR, TON, MTW, FER, CHA, ER1, RHO, GIS, BOG, NON, EMT, HEU, KFI, FAU, DDR, MTT, RUT, RTH, LTL, MAX, GME, NES, OFF, SGT, EUO, MFS, MWW and ITQ-4. It is also possible to use titanium-containing zeolites having the UTD-1, CIT-1 or CIT-5 structure in the process of the present invention. Furthermore, other heterogeneous and homogeneous catalysts may be used. Examples include, but are not limited to, soluble metal catalysts such as ligand-bound rhenium, tungsten, and manganese, along with the heterogenized forms of these.

For the embodiments, the catalyst can be used within a range of from 0.1 wt% to 30 wt%, more preferably within a range of from 0.1 wt% to 15 wt%, and still more preferably within a range of from 0.1 wt% to 5 wt%, based on the total weight of the reaction mixture.

Catalysts used in epoxidations will eventually deactivate. Once the catalyst deactivates, the deactivated catalyst can be separated and regenerated to be reused with a subsequent epoxidation process. The formation of by-products, and in particular the higher molecular weight by-products, can increase the rate of deactivation by plugging the pores of the catalyst. As provided herein, the process for epoxidizing propylene of the present invention helps minimize the amount of by-products formed. Minimizing the by-products reduces the rate at which the pores of the catalyst become plugged. Therefore, the embodiments of the present invention increase the lifetime of the catalyst as compared to an epoxidation of propylene without using the supported base to adjust the pH of the hydrogen peroxide solution to a predetermined pH. Increasing the lifetime of the catalyst can reduce the frequency at which the catalyst needs to be separated and generated, which can reduce the cost associated with the epoxidation process.

For the embodiments, the epoxidation process is carried out in the presence of a solvent. The solvent can be selected from protic solvents. For example, alcohols, such as methanol, ethanol, isopropyl alcohol, tert-butyl alcohol and cyclohexanol, along with ketones, such as acetone, methyl ethyl ketone and acetophenone can be used. The solvent can also be selected from ethers, hydro-alcohol mixtures, aliphatic and aromatic hydrocarbons, halogenated hydrocarbons, and esters. Mixtures of the various solvents may also be used. For the embodiments, the amount of the solvent in the reaction mixture can be within a range of from 3 wt% to 90 wt%, more preferably within a range of from 3 wt% to 50 wt%, and still more preferably within a range of from 3 wt% to 10 wt%, based on the total weight of the reaction mixture.

For the embodiments, the epoxidation process can be carried out in the presence of a co-solvent. The co-solvent can be selected from non-water soluble solvents and include, but are not limited to, linear and cyclic alkanes of C₃-C₁₈, halogenated hydrocarbons, deactivated aromatics, amides, solvents containing nitriles, alcohols, and halogenated alcohols or mixtures thereof. Examples of the co-solvent include, but are not limited to, carbon tetrachloride, propyl chloride, chloroform, dichloromethane, dichloroethane, hexane, octane, decalin, perfluorodecalin, mono or poly-chlorinated benzenes, mono- or poly-brominated benzenes, acetopheonone, benzonitrile, acetonitrile, trichlorotrifluoroethane, trichloroethanol, trifluoroethanol or mixtures thereof. For the embodiments, the co-solvent is preferably 1,2-dichlorobenzene. The co-solvent can be used within in a range of from 5 wt% to 70 wt%, more preferably within a range of from 10 wt% to 50 wt%, and still more preferably within a range of from 10 wt% to 30 wt%.

For the embodiments, the epoxidation process is carried out at a predetermined reaction temperature. For the embodiments, the epoxidation process can be done when the propylene is at a liquid state. For example, a predetermined reaction temperature and pressure where the propylene is at a liquid state can include 50 °C and 11.8 standard atmospheres (atm) (1195.3 kilopascal), but other predetermined reaction temperatures and pressures may be used. In addition, the predetermined reaction temperature can remain at a constant temperature during the epoxidation of propylene. Moreover, for the embodiments, the pressure may be modified during the epoxidation.

For the embodiments, the epoxidation process can be carried out in either a continuous, a semi-continuous or in a batch process. The epoxidation process can also be carried out in at least one batch reactor or at least one continuous reactor, or in a combination of these. For example, the reactor may be selected from, but not limited to, one or more continuous stirred tank reactors, tubular reactors and combinations thereof. Additionally, the reactor may be selected from liquid-liquid contactors, such as a Karr Column.

For the embodiments, the hydrogen peroxide solution at the predetermined pH is added to a pre-reaction solution including propylene, catalyst, solvent and co-solvent, if one is used, to form the reaction mixture. The hydrogen peroxide solution at the predetermined pH can be added to form the reaction mixture with or without the supported base. If desired, the supported base can be removed from the hydrogen peroxide solution by standard separating procedures, such as, but not limited to, vacuum filtration.

For the embodiments, a substantial portion of the produced propylene oxide will reside in an organic phase that can include unreacted propylene, the co-solvent, and a portion of the by-products. However, a portion of the resulting propylene oxide may reside in an aqueous phase that can include unreacted hydrogen peroxide, the solvent, and a portion of the by-products. Thus, the organic and aqueous phase can be separated from the supported base, if not previously removed, and the catalyst by conventional techniques for separation such as decantation, hydrocyclones, mechanically driven high gravity devices or combinations thereof. Additionally, the resulting epoxide can be separated and/or recovered from the organic phase and the aqueous phase using conventional techniques, such as, but limited to, distillation.

### EXAMPLES

The following are examples given to illustrate, but not limit, the scope of this invention.

Synthesis Examples 1 - 10 and Comparative Synthesis Examples A - G are examples for the epoxidation of allyl chloride to epichlorohydrin. The examples for producing propylene oxide are prophetic and illustrated in Examples 1-10. The only difference between Examples 1-10 and Synthesis Examples 1-10 is that the olefin used in Examples 1-10 is propylene instead of allyl chloride. As such, similar results for Examples 1-10 are expected.

### Materials

Hydrogen peroxide solution (30 wt% aqueous solution), available from VWR.

Olefin, allyl chloride (99.4% purity), available from the Dow Chemical Company. The olefin, allyl chloride, is also available from Sigma Aldrich (Reagent Plus, 99%, CAS# 107-05-1).

Olefin, propylene (≥ 99%, CAS# 115-07-1), available from Sigma Aldrich.

Solvent, methanol (99.8%, certified ACS, CAS# 67-56-1), available from Fisher Scientific.

Co-solvent, 1,2-dichlorobenzene (Reagent Plus, 99%, CAS# 95-50-1), available from Sigma Aldrich.

Catalyst, titanium-silicalite (TS-1, titanium content is approximately 2.1 wt%), available from Süd-Chemie.

Supported base, poly-4-vinylpyridine (CAS# 25232-41-1), available from Sigma Aldrich.

Supported base, Amberlyst® A-21 (free base, 20-5- mesh, CAS# 9049-93-8), available from Sigma Aldrich.

Supported base, Lewatit® MP-62 (free base, 300-1000 micrometer (µm)), available from Sigma Aldrich.

Supported base, Dowex® MWA-1 (free base, 53-75 mesh, CAS# 63993-97-9), available from Sigma Aldrich.

Ion exchange resin, Amberlyst® A-26 (hydroxide form, 16-45 mesh, CAS# 39339-85-0, available from Sigma Aldrich.

Ion exchange resin, silica-supported trimethylpropyl ammonium carbonate (0.8 millimole per gram loading, 200-400 mesh), available from Sigma Aldrich.

Ion exchange resin, Reillex® HPQ ion exchange resin (partially quatemized methyl chloride salt, 300-1000 µm particle size, CAS # 125200-80-8), available from Sigma Aldrich,

Homogeneous ionic base, sodium Hydroxide (Reagent grade, ≥ 98%, CAS# 1310-73-2), available from Sigma Aldrich.

### Test Methods

### Measurements of pH

The pH was measured on a Beckman model 45 pH meter using an Orion 8272BN combination electrode with 3M potassium chloride (KCl) filling solution. The Beckman was calibrated daily with pH = 4 and pH = 7 buffers.

### Gas Chromatography

The gas chromatography (GC) was performed on an HP 6890 series G130A GC with a JP 7682 series injector and flame ionization detector.

### Titration

The amount of hydrogen peroxide was analyzed by iodometric titration using 0.01 normality (N) sodium thiosulfate. The hydrogen peroxide concentration was calculated as follows: parts per million (ppm) hydrogen peroxide = (milliliter (ml) titrant used) (0.01 N) (17000)/g sample. Titrations were performed using a Mettler Toledo DL5x V2.3 titrator with a DM140 sensor.

### Synthesis Example 1: Epoxidation of allyl chloride using hydrogen peroxide solution with a supported base

A pre-reaction solution was made by adding 52.3 wt% of allyl chloride, 5 wt% of methanol, 23.3 wt% of 1,2-dichlorobenzene and 1.4 wt% of the TS-1 catalyst to a 750 ml jacketed glass reactor with a stainless steel cooling coil, thermocouple, mechanical stirrer, additional funnel, nitrogen purge with gas scrubber, and reflux condenser/cold finger combination, where the weight percents are based on the total weight of the reaction mixture. The pre-reaction solution was heated to 25.5 °C.

The hydrogen peroxide solution was stirred with enough Amberlyst® A-21 to adjust the pH of the hydrogen peroxide solution to a predetermined pH of 5.7. The amount of hydrogen peroxide solution stirred with the Amberlyst® A-21 was 18 wt% of the total reaction mixture. The mixture of the hydrogen peroxide solution and the Amberlyst® A-21 were added to the addition funnel. The hydrogen peroxide solution and Amberlyst® A-21 mixture were slowly added to the reactor containing the pre-reaction solution forming the reaction mixture. The reaction mixture was heated to the predetermined reaction temperature of 40 °C +/- 0.5 °C for 60 minutes, while being stirred at 600 revolutions per minute (rpm). The contents of the reactor were collected from the reactor into two 250 milliliter (ml) centrifuge tubes and centrifuged at 3000 rpm at 0 °C for 30 minutes. The aqueous phase and the organic phase were decanted into a sepatory funnel. Both phases were analyzed with gas chromatography. The remaining hydrogen peroxide was determined by titration with sodium thiosulfate. The results of Example 1 and the following Examples 2-10 are illustrated in Table 1.

### Synthesis Example 2: Epoxidation of allyl chloride using hydrogen peroxide solution with a supported base

The procedure in Example 1 was repeated, but with the following changes. The hydrogen peroxide solution was stirred with enough poly-4-vinylpyridine to adjust the pH of the hydrogen peroxide solution to a predetermined pH of 5.0.

### Synthesis Example 3: Epoxidation of allyl chloride using hydrogen peroxide solution with a supported base.

The procedure in Example I was repeated, but with the following changes. The hydrogen peroxide solution was stirred with enough Lewatit® MP-62 to adjust the pH of the hydrogen peroxide solution to a predetermined pH of 5.2.

### Synthesis Example 4: Epoxidation of allyl chloride using hydrogen peroxide solution with a supported base

The procedure in Example 1 was repeated, but with the following changes. The hydrogen peroxide solution was stirred with enough Dowex® MWA-1 to adjust the pH of the hydrogen peroxide solution to a predetermined pH of 5.2.

### Synthesis Example 5: Epoxidation of allyl chloride using hydrogen peroxide solution with a supported base

The procedure in Example I was repeated, but with the following changes. The hydrogen peroxide solution was stirred with enough poly-4-vinylpyridine to adjust the pH of the hydrogen peroxide solution to a predetermined pH of 4.5.

### Synthesis Example 6: Epoxidation of allyl chloride using hydrogen peroxide solution with a supported base

The procedure in Example 1 was repeated, but with the following changes. The hydrogen peroxide solution was stirred with Amberlyst® A-21 to adjust the pH of the hydrogen peroxide solution to a predetermined pH of 4.6.

### Synthesis Example 7: Epoxidation of allyl chloride using hydrogen peroxide solution with a supported base

The procedure in Example 1 was repeated, but with the following changes. The pH of the hydrogen peroxide solution was adjusted to a predetermined pH of 5.5. The Amberlyst® A-21 was separated from the hydrogen peroxide solution using vacuum filtration. The addition funnel was charged with the filtered hydrogen peroxide solution (i.e., the filtrate). The filtered hydrogen peroxide solution was then slowly added to the reactor containing the pre-reaction solution.

### Synthesis Example 8: Epoxidation of allyl chloride using hydrogen peroxide solution with a supported base and catalyst reuse

The procedure in Example 7 was repeated, but with the following changes. Instead of using fresh TS-1 for the catalyst, the separated catalyst from Example 7 was reused. The pH of the hydrogen peroxide solution was adjusted to a predetermined pH of 5.6 before vacuum filtration.

### Synthesis Example 9: Epoxidation of allyl chloride using hydrogen peroxide solution with a supported base

The procedure in Example 7 was repeated, but with the following changes. The hydrogen peroxide solution was stirred with enough poly-4-vinylpyridine to adjust the pH of the hydrogen peroxide solution to a predetermined pH of 5.6 before vacuum filtration.

### Synthesis Example 10: Epoxidation of allyl chloride using hydrogen peroxide solution with a supported base and catalyst reuse

The procedure in Example 8 was repeated, but with the following changes. Instead of using fresh TS-1 catalyst, the separated TS-1 catalyst from Example 9 reused. The hydrogen peroxide solution was stirred with enough poly-4-vinylpyridine to adjust the pH of the hydrogen peroxide solution to a predetermined pH of 5.4 before vacuum filtration.

### Comparative Synthesis Examples

The following are Comparative Synthesis Examples to the Synthesis Examples. Comparative Synthesis Examples A-E adjust the pH of the hydrogen peroxide solution with ion exchange resins and homogeneous ionic bases. Comparative Examples F and G do not use any form of pH adjustment or control. The results of the Comparative Synthesis Examples are illustrated in Table 2 and Table 3.

### Comparative Synthesis Example A: Epoxidation of allyl chloride using hydrogen peroxide solution with an ion exchange resin

The procedure from Example 1 was repeated, but with the following changes. The hydrogen peroxide solution was stirred with enough Amberlyst® A-26 to adjust the pH of the hydrogen peroxide solution to 5.5. The results for Comparative Example A and the following Comparative Examples B-E are shown in Table 2. The results for the following Comparative Examples F and G are shown in Table 3.

### Comparative Synthesis Example B: Epoxidation of allyl chloride using hydrogen peroxide solution with an ion exchange resin

The procedure from Example 1 was repeated, but with the following changes. The hydrogen peroxide solution was stirred with enough silica-supported trimethylpropyl ammonium carbonate to adjust the pH of the hydrogen peroxide solution to 5.0.

### Comparative Synthesis Example C: Epoxidation of allyl chloride using hydrogen peroxide solution with an ion exchange resin

The procedure from Example 1 was repeated, but with the following changes. The hydrogen peroxide solution was stirred with enough Reillex® HPQ to adjust the pH of the hydrogen peroxide solution to 5.5.

### Comparative Synthesis Example D: Epoxidation of allyl chloride using hydrogen peroxide solution with a homogeneous ionic base

The procedure from Example 1 was repeated, but with the following changes. The hydrogen peroxide solution was mixed with enough sodium hydroxide aqueous solution to adjust the pH of the hydrogen peroxide solution to 5.6

### Comparative Synthesis Example E: Epoxidation of allyl chloride using hydrogen peroxide solution with a homogeneous ionic base

The procedure from Example 1 was repeated, but with the following changes. The hydrogen peroxide solution was mixed with enough sodium hydroxide aqueous solution to adjust the initial pH of the hydrogen peroxide solution to 6.2. Sodium hydroxide solution was periodically added throughout the reaction to maintain the pH at or above 5.0.

### Comparative Synthesis Example F: Epoxidation of allyl chloride using hydrogen peroxide solution without any form of pH adjustment or control

The procedure from Example 1 was repeated, but with the following changes. The pH of the hydrogen peroxide solution was not adjusted. Therefore, nothing was done to the hydrogen peroxide solution to adjust the pH.

### Comparative Synthesis Example G: Epoxidation of allyl chloride using hydrogen peroxide solution without any form of pH adjustment or control and catalyst reuse

The procedure from Synthesis Example 1 was repeated, but with the following changes. The pH of the hydrogen peroxide solution was not adjusted. Therefore, nothing was done to the hydrogen peroxide solution to adjust the pH. Additionally, instead of using fresh TS-1 catalyst, the separated TS-1 catalyst from Comparative Example F was reused.

**Table 1**

| | **Predetermined pH of the Hydrogen Peroxide Solution** | **Reaction Time (min)** | **Hydrogen Peroxide Conversion** | **Hydrogen Peroxide Utilization** | **CMP/epi** | **MCH/epi** | **Higher Molecular Weight By-products/epi** |
|---|---|---|---|---|---|---|---|
| Synthesis Example 1 | 5.7 | 60 | 99.40% | 93.40% | 1.00% | 0.40% | 0.44% |
| Synthesis Example 2 | 5.0 | 60 | 99.80% | 93.90% | 0.80% | 0.30% | 0.12% |
| Synthesis Example 3 | 5.2 | 60 | 99.40% | 88.70% | 0.90% | 0.30% | 0.21% |
| Synthesis Example 4 | 5.2 | 60 | 97.30% | 90.90% | 1.00% | 0.50% | 0.15% |
| Synthesis Example 5 | 4.5 | 60 | 98.70% | 89.90% | 0.80% | 0.30% | 0.17% |
| Synthesis Example 6 | 4.6 | 60 | 99.50% | 92.60% | 0.80% | 0.30% | 0.17% |
| Synthesis Example 7 | 5.5 | 60 | 99.70% | 86.10% | 0.80% | 0.30% | 0.17% |
| Synthesis Example 8 | 5.6 | 90 | 99.40% | 84.20% | 1.50% | 0.60% | 0.29% |
| Synthesis Example 9 | 5.6 | 60 | 99.70% | 75.20% | 0.90% | 0.30% | 0.17% |
| Synthesis Example 10 | 5.4 | 90 | 99.50% | 74.90% | 1.80% | 0.60% | 0.35% |

**Table 2**

| Comparative Synthesis Example | **Predetermined pH of the Hydrogen Peroxide Solution** | **Reaction Time (min)** | **Hydrogen Peroxide Conversion** | **Hydrogen Peroxide Utilization** | **CMP/epi** | **MCH/epi** | **Higher Molecular Weight By-products/epi** |
|---|---|---|---|---|---|---|---|
| Comparative Synthesis Example A | 5.5 | 60 | 99.70% | 86.10% | 0.90% | 0.30% | 0.12% |
| Comparative Synthesis Example B | 5.0 | 60 | 96.80% | 91.00% | 0.90% | 0.50% | 0.14% |
| Comparative Synthesis Example C | 5.5 | 60 | 99.70% | 76.90% | 1.20% | 0.40% | 0.15% |
| Comparative Synthesis Example D | 5.6 | 60 | 99.50% | 83.40% | 1.00% | 0.40% | 0.20% |
| Comparative Synthesis Example E | 6.2 | 180 | 91.00% | 78.70% | 1.20% | 0.50% | 0.70% |

**Table 3**

| Comparative Synthesis Example | **pH of Reaction Mixture** | **Reaction Time (min)** | **Hydrogen Peroxide Conversion** | **Hydrogen Peroxide Utilization** | **CMP/epi** | **MCH/epi** | **Higher Molecular Weight By-products/epi** |
|---|---|---|---|---|---|---|---|
| Comparative Synthesis Example F | ∼4 | 60 | 99.30% | 96.30% | 1.40% | 0.50% | 0.38% |
| Comparative Synthesis Example G | ∼4 | 120 | 99.00% | 93.60% | 2.70% | 1.00% | 0.57% |

Table 1 is a summary of the Synthesis Examples and Table 2 and Table 3 are a summary of the Comparative Synthesis Examples. In the tables, "CMP" stands for 1-chloro, 3-methoxy, 2-propanol, "MCH" stands for 1-chloro-2,3-propanediaol (monochlorohydrin), and "epi" stands for epichlorohydrin.

The "hydrogen peroxide conversion" is calculated as (the total amount of hydrogen peroxide that reacts during the epoxidation)/(the amount of hydrogen peroxide added to the reaction mixture), "hydrogen peroxide utilization" is calculated as (the amount of hydrogen peroxide converted to epi)/(the amount of hydrogen peroxide that reacts during the epoxidation). The "selectivity" is the (amount of epi formed)/(amount of epi formed plus all by-products). The selectivity is illustrated in Table 1 as the amount of each by-product that is formed.

A factor in the epoxidation of propylene is the pH. Therefore, when comparing the Synthesis Examples with the Comparative Synthesis Examples it is most meaningful to compare ones that are at approximately the same pH. Thus, Synthesis Examples 1-4 are compared to Comparative Synthesis Examples A-D and Synthesis Examples 5-6 are compared to Comparative Synthesis Examples F-G. Also, it is understood by those skilled in the art that a few percent difference in the hydrogen peroxide utilization makes a significant difference in monetary savings as hydrogen peroxide is the most expensive reagent used in the epoxidation process.

When comparing Synthesis Example 1-4 from Table 1 with Comparative Synthesis Examples A-D from Table 2, it can be seen that the Synthesis Examples give better overall results. Particularly, the hydrogen peroxide utilization of Examples 1-4 is maintained at higher levels as compared to the Comparative Synthesis Examples A-D. For example, the benefits of using the supported base to adjust a hydrogen peroxide solution to a predetermined pH (Synthesis Examples 1-4) versus using an ion exchange resin (Comparative Synthesis Examples A-C) or a homogeneous ionic base (Comparative Synthesis Example D) are demonstrated by lower amounts of CMP, MCH and higher molecular weight by-products, and in particular, demonstrated by the high hydrogen peroxide utilization. Comparative Synthesis Examples A-D, illustrate that although the reaction is reacting approximately the same amount of the hydrogen peroxide a significantly less amount is converting to the desired epoxide, which is shown as the hydrogen peroxide utilization.

The benefit of pH control (Synthesis Example 5 and Synthesis Example 6 in Table 1) versus no pH control (Comparative Synthesis Example F and Comparative Synthesis Example G in Table 3) is demonstrated by a significant improvement in the CMP, MCH and higher molecular weight by-products formed during the epoxidation. The Comparative Synthesis Examples F and G with no pH control demonstrate a reduced selectivity. The reduced selectivity can be seen as the amounts of the CMP, MCH and higher molecular by-products are significantly increased as compared to Synthesis Examples 5 and 6, which use pH control.

Figure 1 illustrates the amount of epi formed during the course of the epoxidation of allyl chloride. Figure 1 compares Synthesis Examples 7-10 with Comparative Synthesis Examples F and G. As seen in Figure 1, adjusting the pH of the hydrogen peroxide solution with the supported base Amberlyst® A-21 (Synthesis Example 7) or poly-4-vinylpyridine (Synthesis Example 9) does not decrease the long-term effectiveness of the catalyst when the catalyst is reused (Synthesis Example 8 and Synthesis Example 10).

The following are prophetic examples of the present invention and predicted results. As discussed above, the only difference is that propylene is used as the olefin instead of allyl chloride.

### Example 1 : Epoxidation of propylene using hydrogen peroxide solution with a supported base

Repeat the procedure form Example 1, except use propylene instead of ally chloride, perform the epoxidation at 11.8 atm and heat the reaction mixture to 50 °C.

### Example 2: Epoxidation of Propylene using hydrogen peroxide solution with a supported base

Repeat the procedure in Example 1, except use propylene instead of allyl chloride, stir the hydrogen peroxide solution with enough poly-4-vinylpyridine to adjust the pH of the hydrogen peroxide solution to a predetermined pH of 5, perform the epoxidation at 11.8 atm and heat the reaction mixture to 50 °C.

### Example 3: Epoxidation of propylene using hydrogen peroxide solution with a supported base.

Repeat the procedure in Example 1, except use propylene instead of allyl chloride and stir the hydrogen peroxide solution with enough Lewatit® MP-62 to adjust the pH of the hydrogen peroxide solution to a predetermined pH of 5.2, perform the epoxidation at 11.8 atm and heat the reaction mixture to 50 °C.

### Example 4: Epoxidation of propylene using_hydrogen peroxide solution with a supported base

Repeat the procedure in Example 1, except use propylene instead of allyl chloride and stir the hydrogen peroxide solution with enough Dowex® MWA-1 to adjust the pH of the hydrogen peroxide solution to a predetermined pH of 5.2, perform the epoxidation at 11.8 atm and heat the reaction mixture to 50 °C.

### Example 5: Epoxidation of propylene using hydrogen peroxide solution with a supported base

Repeat the procedure in Example 1, except use propylene instead of allyl chloride and stir the hydrogen peroxide solution with enough poly-4-vinylpyridine to adjust the pH of the hydrogen peroxide solution to a predetermined pH of 4.5, perform the epoxidation at 11.8 atm and heat the reaction mixture to 50 °C.

### Example 6: Epoxidation of propylene using hydrogen peroxide solution with a supported base

Repeat the procedure in Example 1, except use propylene instead of allyl chloride and stir the hydrogen peroxide solution with enough Amberlyst® A-21 to adjust the pH of the hydrogen peroxide solution to a predetermined pH of 4.6, perform the epoxidation at 11.8 atm and heat the reaction mixture to 50 °C.

### Example 7: Epoxidation of propylene using hydrogen peroxide solution with a supported base

Repeat the procedure in Example 1, except stir the hydrogen peroxide solution with enough Amberlyst® A-21 to adjust the pH of the hydrogen peroxide solution to a predetermined pH of 5.5, perform the epoxidation at 11.8 atm and heat the reaction mixture to 50 °C. Separate the hydrogen peroxide solution from the Amberlyst® A-21 by vacuum filtration. Charge the addition funnel with the filtered hydrogen peroxide (i.e., the filtrate). Slowly add the filtered hydrogen peroxide solution to the reactor containing the pre-reaction solution.

### Example 8: Epoxidation of propylene using hydrogen peroxide solution with a supported base and catalyst reuse

Repeat the procedure in Prophetic Example 7, except instead of using fresh TS-1 for the catalyst, use the catalyst from Example 7. Adjust the pH of the hydrogen peroxide solution to a predetermined pH of 5.6 before vacuum filtration, perform the epoxidation at 11.8 atm and heat the reaction mixture to 50 °C.

### Example 9: Epoxidation of allyl propylene using hydrogen peroxide solution with a supported base

Repeat the procedure in Prophetic Example 7, except stir the hydrogen peroxide solution with enough poly-4-vinylpyridine to adjust the pH of the hydrogen peroxide solution to a predetermined pH of 5.6 before vacuum filtration, perform the epoxidation at 11.8 atm and heat the reaction mixture to 50 °C.

### Example 10: Epoxidation of propylene using hydrogen peroxide solution with a supported base and catalyst reuse

Repeat the procedure in Prophetic Example 8, except reuse the separated catalyst from Example 9. Stir the hydrogen peroxide solution with enough poly-4-vinylpyridine to adjust the pH of the hydrogen peroxide solution to a predetermined pH of 5.4 before vacuum filtration, perform the epoxidation at 11.8 atm and heat the reaction mixture to 50 °C.

As discussed above, Examples 1-10 only differ from Synthesis Examples 1-10 in that the olefin used is propylene versus allyl chloride. As such, similar results are expected. For Examples 1-4, it is expected that the selectivity will increase or be maintained at high levels without decreasing the hydrogen peroxide utilization or decreasing the hydrogen peroxide conversion. It is also expected that Examples 1-10 would produce a decreased amount of by-products, including propanediol, methoxypropanols, and higher molecular weight by-products, without decreasing the hydrogen peroxide utilization as compared to an epoxidation of propylene that does not use the supported base to adjust the pH of the hydrogen peroxide solution to the predetermined pH.

Again, the benefit of pH control in Examples 5 and 6 would be expected to produce similar results as Synthesis Example 5 and Synthesis Example 6. For example, it would be expected that Examples 5 and 6 would provide a significant decrease in the formation of propanediol, methoxypropanols, and higher molecular weight by-products as compared to an epoxidation of propylene that did not use the supported base to adjust the pH of the hydrogen peroxide solution to the predetermined pH.

## Claims

1. A process for epoxidizing propylene, the process comprising:
reacting propylene with a hydrogen peroxide solution at a predetermined pH in the presence of a catalyst and a solvent at a predetermined reaction temperature, wherein a pH of the hydrogen peroxide solution is adjusted to the predetermined pH by contacting the hydrogen peroxide solution with a supported base to remove acidic species from the hydrogen peroxide solution, and wherein a selectivity of an epoxidation of propylene is increased without decreasing a hydrogen peroxide utilization or a hydrogen peroxide conversion as compared to an epoxidation of propylene without the supported base to adjust the pH of the hydrogen peroxide solution to a predetermined pH.

2. The process of claim 1, wherein reacting propylene and the hydrogen peroxide solution at the predetermined pH is done in the presence of a co-solvent.

3. The process of any one of the preceding claims, wherein the hydrogen peroxide solution includes a stabilizer, wherein the pH of the hydrogen peroxide solution is adjusted to the predetermined pH as the acidic species are removed from the stabilizer.

4. The process of any one of the preceding claims, wherein the acidic species include an ion, wherein the supported base receives the ion but does not donate an ion to the hydrogen peroxide solution.

5. The process of any one of the preceding claims, wherein the supported base has a predominantly neutral charge prior to adjusting the pH of the hydrogen peroxide solution.

6. The process of any one of the preceding claims, wherein the supported base gains a positive charge in adjusting the pH of the hydrogen peroxide solution to the predetermined pH.

7. The process of any one of the preceding claims, wherein the predetermined reaction temperature remains constant during the epoxidation reaction.

8. The process of any one of the preceding claims, wherein the predetermined pH is within a range of from 3.0 to 8.0.

9. The process of any one of the preceding claims, wherein the predetermined pH is within a range of from 6.0 to 8.0.

## Patentansprüche

1. Ein Verfahren zum Epoxidieren von Propylen, wobei das Verfahren Folgendes beinhaltet:
Reagieren von Propylen mit einer Wasserstoffperoxidlösung bei einem vorbestimmten pH-Wert in der Gegenwart eines Katalysators und eines Lösungsmittels bei einer vorbestimmten Reaktionstemperatur, wobei ein pH-Wert der Wasserstoffperoxidlösung durch das Kontaktieren der Wasserstoffperoxidlösung mit einer Trägerbase, um säurehaltige Spezies aus der Wasserstoffperoxidlösung zu entfernen, an den vorbestimmten pH-Wert angepasst wird, und wobei eine Selektivität einer Epoxidierung von Propylen erhöht wird, ohne eine Wasserstoffperoxidnutzung oder eine Wasserstoffperoxidkonversion zu mindern, im Vergleich mit einer Epoxidierung von Propylen ohne die Trägerbase, um den pH-Wert der Wasserstoffperoxidlösung an einen vorbestimmten pH-Wert anzupassen.

2. Verfahren gemäß Anspruch 1, wobei das Reagieren von Propylen und der Wasserstoffperoxidlösung bei dem vorbestimmten pH-Wert in der Gegenwart eines Hilfslösungsmittels vorgenommen wird.

3. Verfahren gemäß einem der vorhergehenden Ansprüche, wobei die Wasserstoffperoxidlösung einen Stabilisator umfasst, wobei der pH-Wert der Wasserstoffperoxidlösung an den vorbestimmten pH-Wert angepasst wird, während die säurehaltigen Spezies aus dem Stabilisator entfernt werden.

4. Verfahren gemäß einem der vorhergehenden Ansprüche, wobei die säurehaltigen Spezies ein Ion umfassen, wobei die Trägerbase das Ion empfängt, aber kein Ion an die Wasserstoffperoxidlösung abgibt.

5. Verfahren gemäß einem der vorhergehenden Ansprüche, wobei die Trägerbase vor dem Anpassen des pH-Werts der Wasserstoffperoxidlösung eine überwiegend neutrale Ladung aufweist.

6. Verfahren gemäß einem der vorhergehenden Ansprüche, wobei die Trägerbase beim Anpassen des pH-Werts der Wasserstoffperoxidlösung an den vorbestimmten pH-Wert eine positive Ladung erlangt.

7. Verfahren gemäß einem der vorhergehenden Ansprüche, wobei die vorbestimmte Reaktionstemperatur während der Epoxidierungsreaktion konstant bleibt.

8. Verfahren gemäß einem der vorhergehenden Ansprüche, wobei der vorbestimmte pH-Wert innerhalb eines Bereichs von 3,0 bis 8,0 liegt.

9. Verfahren gemäß einem der vorhergehenden Ansprüche, wobei der vorbestimmte pH-Wert innerhalb eines Bereichs von 6,0 bis 8,0 liegt.

## Revendications

1. Un procédé destiné à époxyder du propylène, le procédé comprenant :
la réaction de propylène avec une solution de peroxyde d'hydrogène à un pH prédéterminé en présence d'un catalyseur et d'un solvant à une température de réaction prédéterminée, dans lequel un pH de la solution de peroxyde d'hydrogène est ajusté au pH prédéterminé en mettant la solution de peroxyde d'hydrogène en contact avec une base supportée pour retirer des espèces acides de la solution de peroxyde d'hydrogène, et dans lequel une sélectivité d'une époxydation de propylène est accrue sans décroître une utilisation de peroxyde d'hydrogène ou une conversion de peroxyde d'hydrogène comparée à une époxydation de propylène sans la base supportée pour ajuster le pH de la solution de peroxyde d'hydrogène à un pH prédéterminé.

2. Le procédé de la revendication 1, dans lequel la réaction de propylène et de la solution de peroxyde d'hydrogène au pH prédéterminé se fait en présence d'un co-solvant.

3. Le procédé de l'une quelconque des revendications précédentes, dans lequel la solution de peroxyde d'hydrogène inclut un stabilisateur, le pH de la solution de peroxyde d'hydrogène étant ajusté au pH prédéterminé à mesure que les espèces acides sont retirées du stabilisateur.

4. Le procédé de l'une quelconque des revendications précédentes, dans lequel les espèces acides incluent un ion, la base supportée recevant l'ion mais ne faisant pas don d'un ion à la solution de peroxyde d'hydrogène.

5. Le procédé de l'une quelconque des revendications précédentes, dans lequel la base supportée a une charge prédominamment neutre préalablement au fait d'ajuster le pH de la solution de peroxyde d'hydrogène.

6. Le procédé de l'une quelconque des revendications précédentes, dans lequel la base supportée prend une charge positive lors de l'ajustement du pH de la solution de peroxyde d'hydrogène au pH prédéterminé.

7. Le procédé de l'une quelconque des revendications précédentes, dans lequel la température de réaction prédéterminée demeure constante durant la réaction d'époxydation.

8. Le procédé de l'une quelconque des revendications précédentes, dans lequel le pH prédéterminé se trouve au sein d'une gamme allant de 3,0 à 8,0.

9. Le procédé de l'une quelconque des revendications précédentes, dans lequel le pH prédéterminé se trouve au sein d'une gamme allant de 6,0 à 8,0.
